# EUROPEAN PATENT APPLICATION

(11) **EP 3 929 179 A1**
(43) Date of publication of application: **29.12.2021**
(21) Application number: 20382538.5
(22) Date of filing: 22.06.2020
(51) Int. Cl.: C07D 209/48, A61K 31/4035

(54) **ANTI-INFLAMMATORY COMPOUNDS AND METHODS FOR THEIR MANUFACTURE**

(71) Applicant: BIOHORM, S.L., 08184 Palau-Solità i Plegamans, Barcelona (ES)
(72) Inventor: VILLANUEVA GÓMEZ, Adrián, 08130 Santa Perpetua de Mogoda, Barcelona (ES); LACALLE BALMAÑA, Lorena, 08291 Ripollet, Barcelona (ES); CODOLÀ DUCH, Zoel, 17002 Girona (ES); GARCÍA TORRES, Jasón, 08042 Barcelona (ES)
(74) Representative: Elzaburu S.L.P.

(57) **Abstract**

The present invention relates to an improved process for the preparation of anti-inflammatory compounds, such as N-[2-[(1S)-1-(3-ethoxy-4-methoxyphenyl)-2-methylsulfonylethyl]-1,3-dioxoisoindol-4-yl]acetamide or a salt or solvate thereof and novel polymorphic forms thereof.

## Description

### Field of the invention

The present invention relates to an improved process for the preparation of anti-inflammatory compounds, such as N-[2-[(1S)-1-(3-ethoxy-4-methoxyphenyl)-2-methylsulfonylethyl]-1,3-dioxoisoindol-4-yl]acetamide or a salt or solvate thereof and novel polymorphic forms thereof.

### Background of the invention

Decreasing TNFa levels, increasing cAMP levels, and inhibiting phosphodiesterase 4 (PDE4) constitute valuable therapeutic strategies for the treatment of many inflammatory, infectious, immunological or malignant diseases. These include but are not restricted to septic shock, sepsis, endotoxic shock, hemodynamic shock and sepsis syndrome, post ischemic reperfusion injury, malaria, mycobacterial infection, meningitis, psoriasis, congestive heart failure, fibrotic disease, cachexia, graft rejection, cancer, autoimmune disease, opportunistic infections in AIDS, rheumatoid arthritis, rheumatoid spondylitis, osteoarthritis, other arthritic conditions, Crohn's disease, ulcerative colitis, multiple sclerosis, systemic lupus erythrematosis, ENL in leprosy, radiation damage, and hyperoxic alveolar injury.

Apremilast is a PDE4 inhibitor chemically known as N-[2-[(1S)-1-(3-ethoxy-4-methoxyphenyl)-2-methylsulfonylethyl]-1,3-dioxoisoindol-4-yl]acetamide. It is indicated for the treatment of adult patients with moderate to severe plaque psoriasis, active psoriatic arthritis or oral ulcers associated with Behçet's disease and available as oral tablets under the trade name of OTEZLA^{®}.

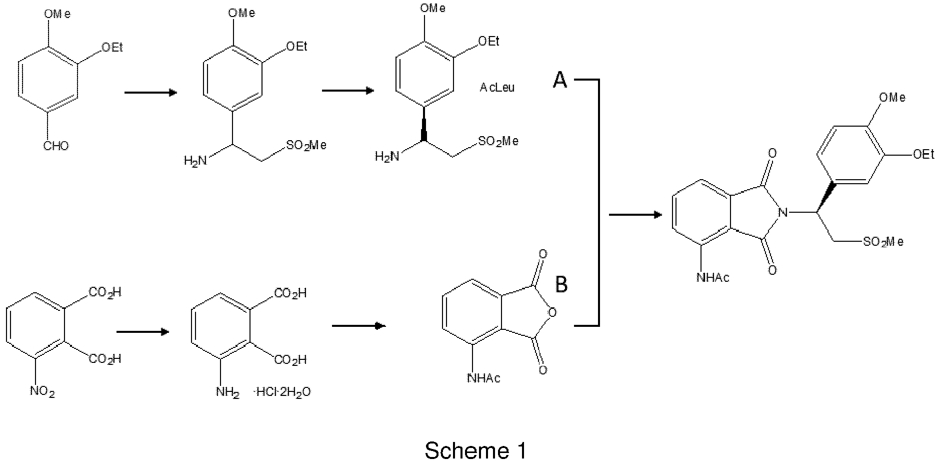

Apremilast was first described in WO0025777. An improved process for its preparation was then described in EP2420490. Starting from 3-nitropthalic acid and 3-ethoxy-4-methoxybenzaldehyde and after several chemical transformations, the two derivatives A (amine) and B (anhydride)are reacted to yield apremilast (Scheme 1).

However, this approach has several disadvantages. Apremilast can be obtained only in moderate yields, and chemical stability as well as analytical difficulties have been encountered. Hence, there is a need to find an alternative route which solves these problems.

Several documents disclose apremilast and crystalline forms thereof and of its salts or solvates. WO2003080049 discloses a stereomerically pure salt of (S)-2- (3-ethoxy-4-methoxyphenyl)-1- (methylsulphonyl)-eth-2-ylamine, and chiral amino acid salts thereof. WO2009120167 discloses several crystalline forms of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide and methods for their preparation. The non-solvated anhydrous forms A, B and F are disclosed, as well as several solvates with organic solvents selected from toluene (form C), methylene chloride (form D), acetonitrile (form E), ethyl acetate (form G) and tetrahydrofuran (form H). WO2016097156 discloses crystalline ethanol, n-propanol, acetone and methyl ethyl ketone solvates of apremilast. WO2017033116 discloses a crystalline p-xylene hemisolvate of apremilast and a dimethyl carbonate solvate of apremilast. WO2017085568 discloses a crystalline benzyl alcohol solvate of apremilast.

While apremilast crystalline form B has been recently reported to have stability issues, most of the apremilast crystalline forms mentioned above have toxicity issues because they contain solvents that must be used in very limited amounts, if any, in pharmaceutical products. Additionally, the disclosed crystalline apremilast solvates consist of very small particles, which makes pharmaceutical development of a drug product challenging due to poor powder characteristics.

The crystalline apremilast solvates described herein are characterized especially by high stability and are easy to prepare. In addition, they are excellent in high crystallinity, which makes them very suitable for the preparation of highly pure apremilast.

### Summary of the invention

In a first aspect, the present invention relates to a process for manufacturing the compound of formula I or a salt or solvate thereof, 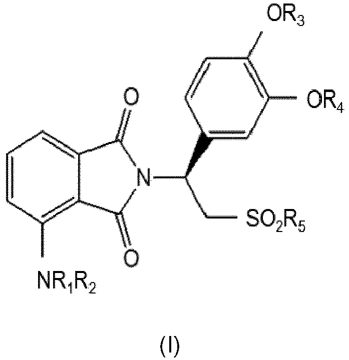 wherein
R₁ and R₂ are independently selected from H, C₁-C₈ alkyl, C₃-C₆ cycloalkyl, aryl or C=OR₆ wherein R₆ is independently selected from H, C₁-C₈ alkyl, C₃-C₆ cycloalkyl, and aryl,
R₃ and R₄ are independently selected from H, C₁-C₈ alkyl, C₃-C₆ cycloalkyl, and aryl,
R₅ is C₁-C₈ alkyl, C₃-C₆ cycloalkyl, and aryl,
the process comprising the following steps:
(a) reacting the compound of formula II or a salt thereof 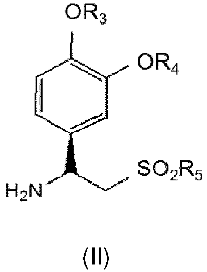 with the compound of formula III.

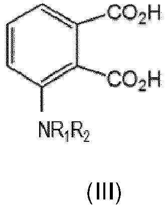

A second aspect of the present invention relates to a pharmaceutical composition comprising the compound of formula (I) or any of the solvates obtained by the process of the first aspect and at least one pharmaceutically acceptable excipient.

A third aspect of the present invention relates to a crystalline anisaldehyde solvate of apremilast, having an X-ray powder diffractogram comprising at least the peaks at diffraction 2-theta angle 7.40, 11.15 and 26.10° 2θ ± 0.2° 2θ.

A fourth aspect of the present invention relates to a crystalline 3-phenylpropanaldehyde solvate of apremilast, having an X-ray powder diffractogram comprising at least the peaks at diffraction 2-theta angle 7.31, 11.08, 25.96 and 11.75° 2θ ± 0.2° 2θ.

A fifth aspect of the present invention relates to a crystalline 3-methyl-2-cyclohexenone solvate of apremilast, having an X-ray powder diffractogram comprising at least the peaks at diffraction 2-theta angle 7.41, 11.17, 26.16 and 17.61 ° 2θ ± 0.2° 2θ.

A sixth aspect of the present invention relates to a crystalline 6-methyl-5-hepten-2-one solvate of apremilast, having an X-ray powder diffractogram comprising at least the peaks at diffraction 2-theta angle 11.25, 7.40, 26.10, 17.61 and 17.80° 2θ ± 0.2° 2θ.

### Description of the invention

The present invention provides an improved process for the manufacture of the compound of formula (I) without the drawbacks of the prior art synthetic processes, which proved to be inefficient and expensive. The present invention provides a shorter process where the compound of formula (III) can be manufactured in a one-pot process, avoiding the use of 3-acetamidophthalic anhydride therefore avoiding analytical problems associated with this compound. Additionally, the reaction solvent may contain or may be water. The process of the present invention comprises the coupling of compounds of formula (II) and (III) for obtaining the compound of formula (I), which is simple, efficient and environmental-friendly.

In a preferred embodiment of the process of the first aspect, R₁ and R₂ are independently selected from H, C₁-C₄ alkyl, and C₃-C₆ cycloalkyl, aryl or C=OR₆ wherein R₆ is independently selected from H, and C₁-C₄ alkyl, C₃-C₆ cycloalkyl, and aryl; R₃ and R₄ are independently selected from H, and C₁-C₄ alkyl, C₃-C₆ cycloalkyl, and aryl; and R₅ is C₁-C₈ alkyl.

As used herein, the term "alkyl" refers to radical linear or branched hydrocarbon chains having 1 to 8 carbon atoms, preferably 1 to 4 carbon atoms, and which bind to the rest of the molecule by a single bond, such as but not limited to, methyl, ethyl, n-propyl, i-propyl, n-butyl, terc-butyl, sec-butyl, n-pentyl, etc. The alkyl groups may be optionally substituted in any of their positions by one or more substituents such as halo, hydroxyl, alkoxyl, carboxyl, carbonyl, cyano, acyl, alkoxycarbonyl, amino, nitro, mercapto and alkylthio.

As used herein, the term "aryl" refers to single or multiple aromatic rings, which have between 5 and 10 carbon atoms, such as but not limited to, phenyl, naphthyl, indenyl, phenantryl,

Preferably the aryl group has 6 to 9 carbon atoms and more preferably the aryl group is a phenyl. The aryl groups may optionally be substituted in any of their positions by one or more substitutes such as halo, hydroxyl, alkoxyl, carboxyl, carbonyl, cyano, acyl, alkoxycarbonyl, amino, nitro, mercapto and alkylthio.

As used herein, the term "cycloalkyl" refers to a stable monocyclic or bicyclic radical with 3 to 10 members, preferably from 3 to 8 members, and more preferably from 3 to 6 members, that is saturated or partially saturated and consists solely of carbon and hydrogen atoms, such as cyclopropyl, cyclobutyl, cyclopentyl, cyclohexyl and adamantyl, and that may be optionally substituted by one or more groups such as halo, hydroxyl, alkoxyl, carboxyl, carbonyl, cyano, acyl, alkoxycarbonyl, amino, nitro, mercapto and alkylthio.

In a further embodiment, R₁ and R₂ are independently selected from H, C₁-C₄ alkyl, and C=OR₆ wherein R₆ is C₁-C₄ alkyl; R₃ and R₄ are independently selected from H and C₁-C₄ alkyl; and R₅ is C₁-C₄ alkyl.

In a further preferred embodiment, R₁ is H, R₂ is acetyl, R₃ is methyl, R₄ is ethyl and R₅ is methyl.

In a preferred embodiment, the compound of formula (II) is in form of the free base or a salt. When the compound of formula (II) is a salt, the salt is preferably selected from (R,R)-4-clorotartranilic acid, (R,R)-di-p-toluoyl-tartaric acid, L-phenylanaline, p-toluenesulfonamide and N-acetyl-L-leucine, preferably is the N-acetyl-L-leucine salt.

In a preferred embodiment of the process of the first aspect, compound III is activated by a coupling agent, preferably an acid activating agent, more preferably selected from carbonyldiimidazole (CDI), thionyl chloride, dicyclohexyl carbodiimide (DCC), 1-Ethyl-3-(3-dimethylaminopropyl)carbodiimide (EDC), hydroxybenzotriazole (HOBt), Hexafluorophosphate Benzotriazole Tetramethyl Uronium (HBTU), Hexafluorophosphate Azabenzotriazole Tetramethyl Uronium (HATU), 6-chloro-1-((dimethylamino)(morpholino)-methylene)-1 H-benzotriazolium hexafluorophosphate 3-oxide (HDMC), more preferably selected from carbonyldiimidazole (CDI), thionyl chloride, dicyclohexyl carbodiimide (DCC), 1-Ethyl-3-(3-dimethylaminopropyl)carbodiimide (EDC), and Hexafluorophosphate Azabenzotriazole Tetramethyl Uronium (HATU), and even more preferably is activated by CDI.

In a preferred embodiment of the process of the first aspect, the solvent is an organic solvent, preferably selected from acetonitrile (ACN), dichloromethane (DCM), tetrahydrofuran (THF), 2-methyltetrahydrofuran, toluene and ethanol, more preferably is ACN.

In a preferred embodiment of the process of the first aspect, step (a) is performed under heating to reflux.

In a preferred embodiment of the process of the first aspect, the process further comprises step (b) precipitating the compound of formula I. Preferably, compound I precipitates from a mixture of the organic solvent, preferably ACN, and water, more preferably from a mix of about 1:1 to 10:1 of ACN and water, preferably from a mix of about 3:1 to 9:1, preferably from a mix of about 4:1 to 8:1, even more preferably from a mix of about 6:1 mix of ACN and water.

In a preferred embodiment of the process of the first aspect, compound III is synthesized from compound IV.

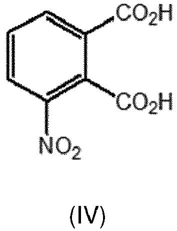

In a preferred embodiment of the process of the first aspect, compound IV is transformed into compound V before transforming into compound III.

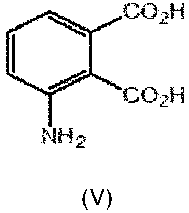

Compound V may be isolated as a salt, such as a hydrochloride dihydrate salt, but preferably compound V is only formed *in situ* and transformed directly, without isolating, into compound III.

The process of the present invention can be seen in Scheme 2 below:

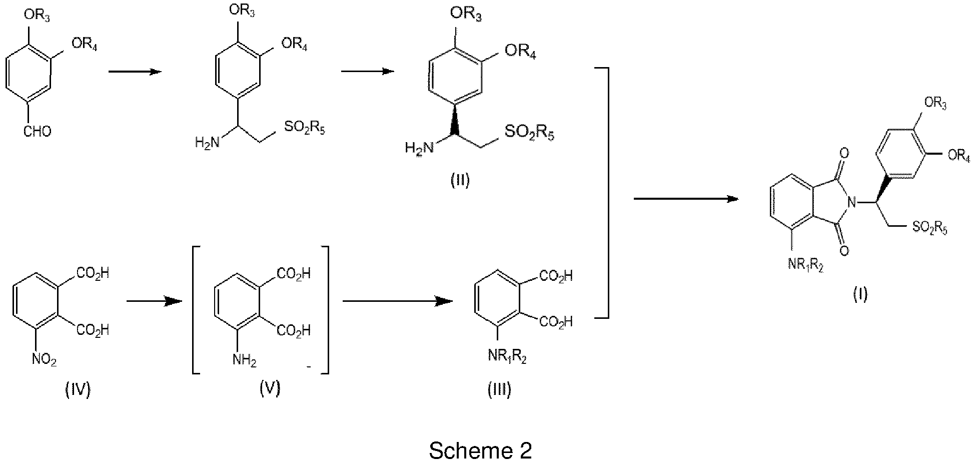

In a preferred embodiment, the process of the present invention is depicted in Scheme 3:

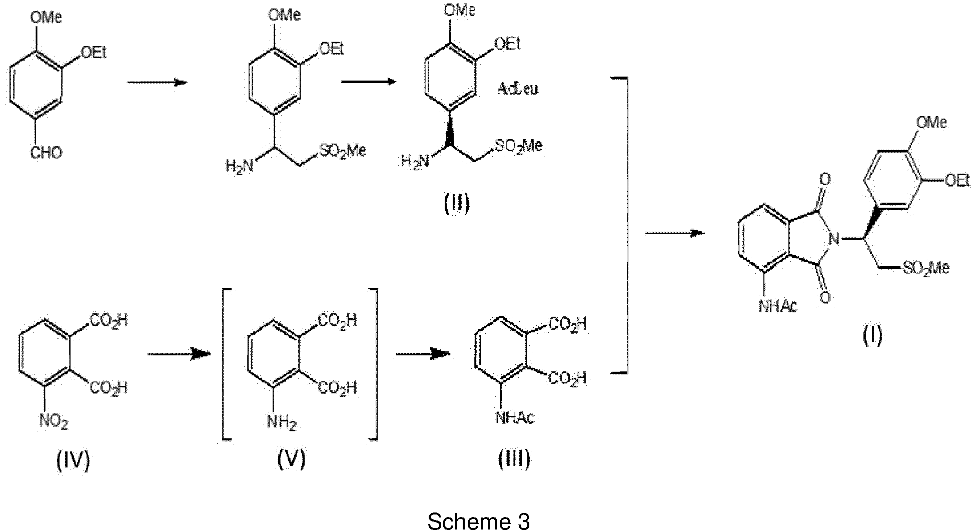

In a preferred embodiment, the compound of formula (I) is obtained by reacting the compound of formula (II) and the compound of formula (III) as in Scheme 3 using CDI in ACN to yield apremilast with 20 % more yield than using the process of Scheme 1. Also, the use of ACN as solvent in the coupling reaction allows softer reaction conditions and easier work-up during product isolation.

Another advantage of the process of the present invention is that compound of formula (III) has an increased half-life stability over of 3-acetamidophthalic anhydride used in the process of Scheme 1, obtaining a product with higher purity. Under the prior art conditions, compound II may dimerize. This is avoided under the reaction conditions of the present invention. The process of the invention permits an overall cost reduction by lowering the catalytic charge in the synthesis of the compound of formula (III) and the use of cheaper solvents. Another advantage of the process of the invention is that the amount of reaction by-products/impurities is reduced to a minimum when the compound of formula (III) is used, because the use of 3-acetamidophthalic anhydride led to the formation of ring-opened apremilast impurities, and the use of water as the solvent instead of alcohol and acetic anhydride, as in the prior art ,permits avoiding the formation of salts as subproducts, reducing the final impurities. Using water as solvent has the advantage that salts are removed, and this allows using reducing agents like metal hydrides or hydrogen generating agents to synthetize comp. III, instead of using H₂, which requires expensive autoclaves and high safety measures due to the elevated pressures required, making this reaction also more environmentally friendly and more sustainable.

In a preferred embodiment of the process of the first aspect, the process comprises the use of a catalyst and a reducing agent, the catalyst being selected from Pd, Pt, Rh, Ru, Raney nickel, Fe in HCI, Sn on HCI or Zn in HCl; and the reducing agent being preferably selected from formic acid, formates selected from ammonium, potassium and sodium formates, H₂, sodium borohydride, polimethylhidrosiloxane (PMHS) and triethylsilane (TES). In a more preferred embodiment of the process of the first aspect, the process comprises the use of a catalyst and a reducing agent, the catalyst being preferably a heterogenic catalyst selected from Pd on C, Pt on C, Rh on C, or Ru on C; and the reducing agent being preferably selected from formic acid, formates selected from ammonium, potassium and sodium formates, H₂, sodium borohydride, polimethylhidrosiloxane (PMHS) and triethylsilane (TES).

In a preferred embodiment of the process of the first aspect, the amount of catalyst is from about 1 to 10 % by weight, preferably from about 1 to 8 % by weight of catalyst, more preferably from about 1.5 to 5 % by weight of catalyst, and even more preferably from about 1.5 to 2.5 % by weight of catalyst in respect of the weight of compound IV. In a preferred embodiment, the amount of catalyst is about 2 % by weight in respect of the weight of compound IV.

In a preferred embodiment of the process of the first aspect, formic acid is used as reducing agent and Pd is used as catalyst, preferably Pd/C.

In a preferred embodiment of the process of the first aspect, compound III synthesis is carried out at a temperature below 60 °C, preferably below 50 °C, more preferably between 35 and 45 °C.

In a preferred embodiment of the process of the first aspect, the process further comprises providing a solution of the compound of formula I in a solvent selected from anisaldehyde, 3-phenylpropanaldehyde, 3-methyl-2-cyclohexenone and 6-methyl-5-hepten-2-one and isolating the respective solvate of the compound of formula I.

Another aspects of the present invention are a crystalline anisaldehyde solvate of apremilast, a crystalline 3-phenylpropanaldehyde solvate of apremilast, a crystalline 3-methyl-2-cyclohexenone solvate of apremilast, or a crystalline 6-methyl-5-hepten-2-one solvate of apremilast obtainable by the process of the first aspect.

In a second aspect, the present invention relates to a pharmaceutical composition comprising the compound of formula (I) or any of the solvates obtained by the process of the first aspect and at least one pharmaceutically acceptable excipient. In a preferred embodiment, the pharmaceutical composition of the second aspect comprises apremilast or a crystalline 3-phenylpropanaldehyde solvate of apremilast, a crystalline 3-methyl-2-cyclohexenone solvate of apremilast, or a crystalline 6-methyl-5-hepten-2-one solvate of apremilast, and is in form of a tablet, preferably a film-coated tablet. In a preferred embodiment, the pharmaceutical composition of the second aspect comprises at least one of the crystalline solvents of apremilast of the third to sixth aspects of the invention.

The term "pharmaceutically acceptable" indicates that the substance or composition must be compatible chemically and/or toxicologically, with the other ingredients comprising a formulation, the mammal being treated therewith, and/or the route of administration of the composition. The pharmaceutical composition of the second aspect my comprise as pharmaceutically acceptable excipient a diluent, a disintegrant, a lubricant, etc. The term "diluent" as used herein refers to pharmaceutically acceptable excipients which are added to the bulk volume of the active agent making up the solid composition. As a result, the size of the solid composition increases, which makes its size suitable for handling. Diluents are convenient when the dose of drug per solid composition is low and the solid composition would otherwise be too small. The diluent may be selected for example from microcrystalline cellulose or lactose monohydrate. The disintegrant may be selected for example from croscarmellose sodium or crospovidone. As used herein, "disintegrant" means a substance or a mixture of substances added to a tablet to facilitate its breakup or disintegration after administration. The lubricant may be for example magnesium stearate. As used herein, "lubricant" means a substance that reduces friction between the composition of the present invention and the surfaces of the apparatus used to compact the composition into a compressed form.

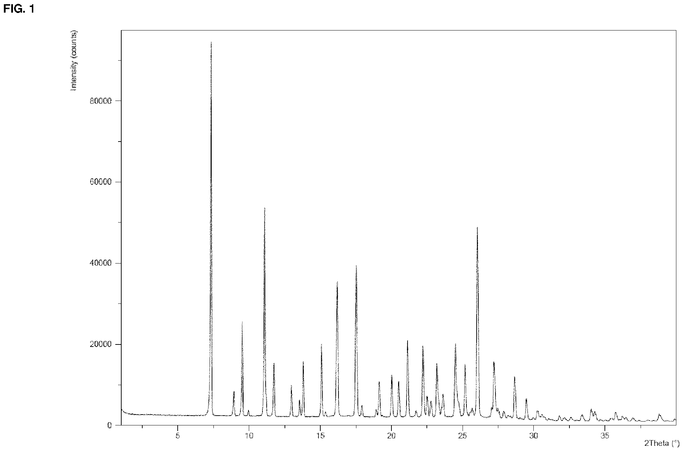

A third aspect of the present invention relates to a crystalline anisaldehyde solvate of apremilast having an X-ray powder diffractogram comprising at least the peaks at diffraction 2-theta angle 7.40, 11.15 and 26.10° 2θ ± 0.2° 2θ. Preferably, the X-ray powder diffractogram comprises at least the peaks at diffraction 2-theta angle 7.40, 11.15, 26.10, 17.59 and 16.25° 2θ ± 0.2° 2θ. More preferably, the X-ray powder diffractogram comprises at least the peaks at diffraction 2-theta angles 7.40, 8.99, 9.58, 10.02, 11.15, 11.81, 13.05, 13.62, 13.87, 15.15, 15.44, 16.25, 17.06, 17.59, 18.00, 19.00, 19.20, 20.09, 20.58, 21.21, 21.82, 22.28, 22.58, 22.86, 23.25, 23.72, 24.55, 24.83, 25.23, 25.40, 25.60, 25.75, 26.10, 27.09, 27.28, 27.59, 27.96, 28.24, 28.45, 28.72, 29.09, 29.53, 30.04, 30.29, 30.65, 30.84, 31.10, 31.40, 31.85, 32.27, 32.65, 33.06, 33.39, 33.51, 34.09, 34.34, 34.74, 35.45, 35.82, 36.29, 36.54, 37.02, 37.42, 38.04, 38.49 and 38.86° 2θ ± 0.2° 2θ, also shown in figure 1.

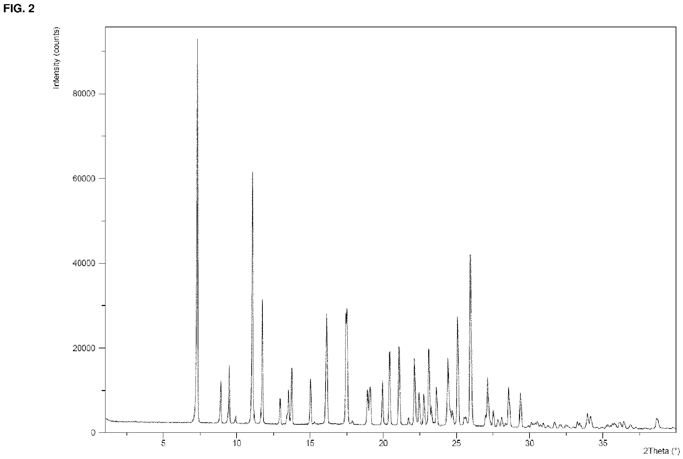

A fourth aspect of the present invention relates to a crystalline 3-phenylpropanaldehyde solvate of the compound of formula I, having an X-ray powder diffractogram comprising at least the peaks at diffraction 2-theta angle 7.31, 11.08, 25.96 and 11.75° 2θ ± 0.2° 2θ. Preferably, X-ray powder diffractogram comprises at least the peaks at diffraction 2-theta angle 7.31, 11.08, 25.96, 11.75, 16.14, 17.47 and 25.08° 2θ ± 0.2° 2θ. More preferably, the X-ray powder diffractogram comprises at least the peaks at diffraction 2-theta angles 7.31, 8.91, 9.49, 9.93, 11.08, 11.75, 12.96, 13.55, 13.76, 15.04, 15.33, 16.14, 17.47, 17.90, 18.92, 19.10, 19.96, 20.44, 21.10, 21.74, 22.14, 22.45, 22.78, 23.12, 23.65, 24.41, 24.73, 25.08, 25.52, 25.64, 25.96, 26.96, 27.13, 27.51, 27.84, 28.09, 28.35, 28.57, 29.36, 30.11, 30.37, 30.53, 30.74, 30.94, 31.21, 31.69, 32.03, 32.48, 32.96, 33.24, 33.44, 33.94, 34.16, 34.55, 35.32, 35.64, 35.77, 36.14, 36.42, 36.87, 37.23, 37.85, 38.66 and 39.74° 2θ ± 0.2° 2θ, also shown in figure 2.

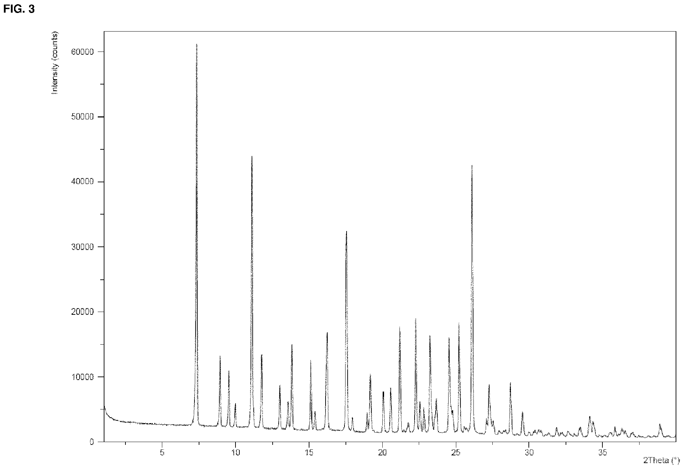

A fifth aspect of the present invention relates to a crystalline 3-methyl-2-cyclohexenone solvate of the compound of formula I, having an X-ray powder diffractogram comprising at least the peaks at diffraction 2-theta angle 7.41, 11.17, 26.16 and 17.61° 2θ ± 0.2° 2θ. Preferably, the X-ray powder diffractogram comprises at least the peaks at diffraction 2-theta angle 7.41, 11.17, 26.16, 17.61, 22.33, 24.60, 21.21, 16.29, 25.29 and 23.30° 2θ ± 0.2° 2θ. More preferably, the X-ray powder diffractogram comprises at least the peaks at diffraction 2-theta angles 7.41, 9.00, 9.60, 10.04, 11.16, 11.82, 13.07, 13.63, 13.90, 15.19, 15.47, 16.29, 17.61, 18.02, 19.02, 19.24, 20.13, 20.62, 21.24, 21.52, 21.84, 22.33, 22.62, 22.88, 23.30, 23.73, 24.60, 24.85, 25.29, 25.63, 26.16, 27.15, 27.34, 27.61, 28.01, 28.30, 28.45, 28.77, 29.23, 29.60, 30.04, 30.45, 30.70, 30.86, 31.38, 31.91, 32.32, 32.68, 33.11, 33.50, 34.15, 34.42, 34.82, 35.17, 35.53, 35.87, 36.15, 36.36, 36.59, 37.11, 37.51, 38.13, 38.51, 38.96 and 39.55° 2θ ± 0.2° 2θ, also shown in figure 3.

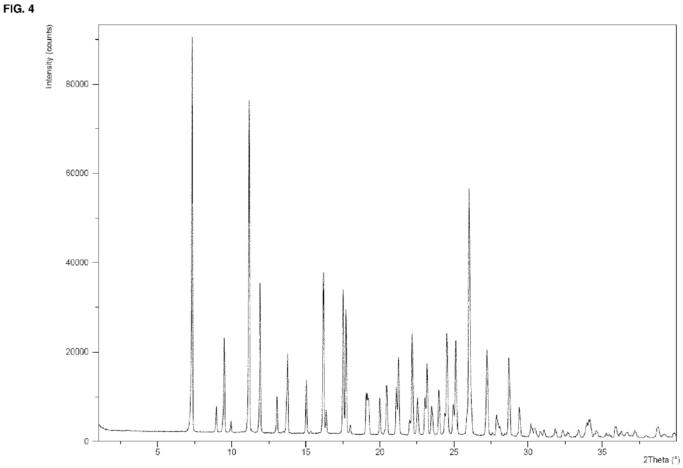

A sixth aspect of the present invention relates to a crystalline 6-methyl-5-hepten-2-one solvate of the compound of formula I, having an X-ray powder diffractogram comprising at least the peaks at diffraction 2-theta angle 11.25, 7.40, 26.10, 17.61 and 17.80° 2θ ± 0.2° 2θ. Preferably, the X-ray powder diffractogram comprising at least the peaks at diffraction 2-theta angle 11.25, 7.40, 26.10, 11.99, 16.27, 17.61 and 17.80° 2θ ± 0.2° 2θ. More preferably, the X-ray powder diffractogram comprises at least the peaks at diffraction 2-theta angles 7.40, 9.04, 9.56, 10.02, 11.25, 11.99, 13.13, 13.85, 15.12, 16.27, 16.46, 17.61, 17.80, 18.09, 19.15, 20.08, 20.55, 21.19, 21.35, 22.09, 22.27, 22.64, 23.12, 23.28, 23.58, 24.07, 24.62, 25.03, 25.22, 25.92, 26.10, 27.32, 27.68, 27.97, 28.21, 28.80, 29.50, 30.28, 30.52, 30.92, 31.17, 31.91, 32.43, 32.78, 33.49, 33.98, 34.28, 34.55, 34.69, 35.38, 35.60, 35.95, 36.06, 36.41, 36.81, 37.27, 38.08, 38.80 and 39.28° 2θ ± 0.2° 2θ, also shown in figure 4.

### Brief description of the drawings

**Figure 1****.** XRPD pattern of the anisaldehyde solvate of apremilast.
**Figure 2****.** XRPD pattern of the 3-phenylpropanaldehyde solvate of apremilast.
**Figure 3****.** XRPD pattern of the 3-methyl-2-cyclohexenone solvate of apremilast.
**Figure 4****.** XRPD pattern of the 6-methyl-5-hepten-2-one solvate of apremilast.
**Figure 5****.** Thermogravimetric analysis of the anisaldehyde solvate of apremilast.
**Figure 6****.** Thermogravimetric analysis of the 3-phenylpropanaldehyde solvate of apremilast.
**Figure 7****.** Thermogravimetric analysis of the 3-methyl-2-cyclohexenone solvate of apremilast.
**Figure 8****.** Thermogravimetric analysis of the 6-methyl-5-hepten-2-one solvate of apremilast.

### Examples

The following examples are provided to further illustrate, but not to limit this invention.

Analytical measurements have been conducted as follows:
1) Analytical conditions for thermogravimetric analysis (TGA):
- Equipment: Mettler Toledo Thermobalance TGA/SDTA851e.
- Crucible: Alumina crucible with a capacity of 70 µL.
- Gas: Dry nitrogen 50 mL/min.
- Method: Heating from 30°C to 300°C at a rate of 10°C/min. A blank curve has been previously performed by using the same methodology and it has been subtracted.
2) Analytical conditions for Powder X-Ray Diffraction (PXRD) analysis:
Sample preparation :the powder samples were sandwiched between films of polyester of 3.6 microns of thickness.
Equipment: *PANalytical X'Pert PRO MPD* q/q powder diffractometer of 240 millimetres of radius, in a configuration of convergent beam with a focalizing mirror and a transmission geometry with flat samples sandwiched between low absorbing films.
   - Cu Ka radiation (I = 1.5418 Å).
   - Work power: 45 kV - 40 mA.
   - Incident beam slits defining a beam height of 0.4 millimetres.
   - Incident and diffracted beam 0.02 radians *Soller* slits.
   - *PIXcel* detector: Active length = 3.347º.
   - 2q/q scans from 1 to 40º 2q with a step size of 0.026º 2q and a measuring time of 300 seconds per step.
Data acquisition: Powder diffraction pattern were acquired on a Bruker D8 Advance Series 2Theta/Theta powder diffraction system using CuKα1-radiation in transmission geometry. The system is equipped with a VÅNTEC-1 single photon counting PSD, a Germanium monochromator, a ninety positions auto-changer sample stage, fixed divergence slits and a radial soller.
Programs used: Data collection with DIFFRAC plus XRD Commander V.2.5.1, and evaluation with Diffrac.EVA V.5.0.0.22.
   - Measurement conditions: The samples were measured in a range from 4 to 40º in 2θ in a 1 hour measurement using an angular step of 0.026º and a time per step of 300 s.
3) Analytical conditions for High Purity Liquid Cromatography (HPLC) analysis:
Reactants: TFA (trifluoroacetic acid) (analysis quality); water, HPLC quality; acetonitrile, HPLC quality
Column: XBridge C18 3,5 µ (150 x 4.6 mm)
∘ Mobile phase: ACN:H2O (TFA 0.05%)
∘ Flow: 1.0 mL/min
∘ Mobile phase gradient:

| t (minutes) | % acetonitrile | % aqueous phase |
|---|---|---|
| 0.0 | 5 | 95 |
| 3.0 | 5 | 95 |
| 20.0 | 80 | 20 |
| 25.0 | 80 | 20 |
| 30.0 | 5 | 95 |

- Column temperature: 30°C
- Wavelength: 230 nm
- Injection volume: 10 µL
Aqueous phase TFA 0.05%
Solvent: ACN/0.05% TFA (H2O) (40:60)
Samples: 10 mg of Compound I in 50 mL of solvent

### Synthesis of a compound of formula III (acetaminophthalic acid)

A 250 mL round bottom flask was charged with nitrophthalic acid (a compound of formula IV) (5 g, 23.7 mmols), palladium on charcoal (0.23 g of Pd/C, moisture content 55.8%) and NaOH 3M (50 mL, 10 vol). The mixture was heated to 35-40 °C and pH adjusted to 10 by careful addition of HCOOH (ca. 3.7 mL). The reaction mixture was stirred for 4h and afterwards, Ac₂O (3.91mL, 1.75 eq) was carefully added at 35-40 °C for 30 minutes. The reaction mixture was stirred for additional 30 minutes and cooled to room temperature. The catalyst was filtered off and the product was precipitated by the addition of HCI (37%) to pH <1. The mixture was stirred for 2 h at 0-5 °C, filtered, washed with HCI 1M and dried under vacuum at 40 °C. Acetaminophthalic acid (a compound of formula III) was obtained with good yield (80-85%) and high purity (>95% by HPLC).

### Synthesis of a compound of formula I (apremilast)

A 1 L jacketed reactor equipped with mechanical stirring, temperature probe and condenser was charged with APR-1C (15.74 g, 70.54 mmols, 1.05 eq) and ACN (300 mL, 10 RV). 1,1-carbonyldimidazole (21.79 g, 2 eq) was charged portion wise at room temperature while stirring. The reaction was stirred for 15 min and acetaminophthalic acid (a compound of formula III) (30 g, 67.18 mmol) was charged into the reactor. The mixture was heated to reflux for 3h. Then, 50% of ACN was distilled and the mixture was cooled down to 20-25 °C. Water (150 mL, 5 RV) was added dropwise during 1h. The mixture was seeded with apremilast and stirred for 45 min. Then, water (150 mL, 5 RV) was added dropwise during 1h and aged for 2 h. The mixture was filtered off, and the cake washed with water (30 mL, 1 RV). The wet solid was suspended in EtOH (225 mL, 7.5 RV), and heated up to reflux. After stirring for 10 min, the mixture was filtered, cooled down to RT and aged for 2h. The slurry was filtered, and the cake washed with EtOH (30 mL, 1RV). The final solid was dried at 40 °C, under vacuum to obtain 13.0 g of apremilast (yield 80 %, ee 99.5%).

### Apremilast co-solvates synthesis

Once the final apremilast was achieved following the previous procedure, a series of co-solvates were synthesised following the next procedure: apremilast was charged into a 50mL round-bottom flask, the corresponding solvent was added and the mixture was stirred at the desired temperature for a few hours (see table 1). Once the reaction was completed, the reaction was cooled down and the solvent was filtered. All solids were dried under vacuum at 45 °C until constant weight.

The following table shows apremilast co-solvates reaction conditions:

| **Solvate** | **Volume (RV)** | **Temperature (°C)** | **Time (h)** |
|---|---|---|---|
| Anisaldehyde | 2.00 | 75 | 2 |
| 3-phenylpropanaldehyde | 1.25 | 75 | 2 |
| 3-methyl-2-cyclohexenone | 5.00 | 25 | 2 |
| 6-methyl-5-hepten-2-one | 2.00 | 80 | o/n |

| | | | |
|---|---|---|---|
| RV: relative volume to apremilast volume. o/n: overnight. | | | |

All four apremilast co-solvates were analysed, and their stability was tested.

Stability testing was performed at the conditions of 40 ºC and 75% relative humidity (r.h.) and showed that all four solvates remained unchanged over 6 months at these extreme conditions. Hence, all four solvates show excellent stability.

### Synthesis of an anisaldehyde solvate of a compound of formula I (apremilast)

Apremilast (12g, 26mmol) and anisaldehyde (24 mL, 2.0 RV) were charged into a 50mL round-bottom flask, and the mixture was stirred at 75-80ºC for a 3h. Once the reaction was completed, the reaction was cooled down to RT and then to 0-5ºC and stirred for 2h. The product was filtered and washed with cyclohexane. The solid was dried under vacuum at 45 °C until constant weight, to obtain 10.59 g of the product.

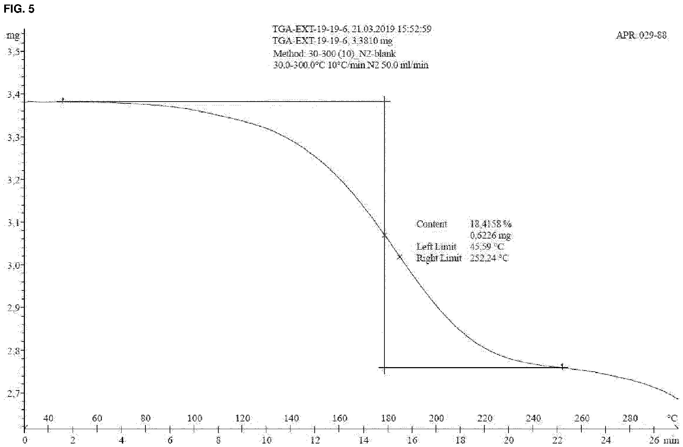

The product was characterized by TGA and PXRD as it can be seen in figures 1 and 5.

### Synthesis of a 3-phenylpropanaldehyde solvate of a compound of formula I (apremilast)

Apremilast (12g, 26mmol) and 3-phenylpropanaldehyde (15 mL, 1.25 RV) were charged into a 50mL round-bottom flask, and the mixture was stirred at 75-80ºC for a 3h. Once the reaction was completed, the reaction was cooled down to RT and then to 0-5ºC and stirred for 2h. The product was filtered and washed with cyclohexane. The solid was dried under vacuum at 45 °C until constant weight, to obtain 13.96 g of the product.

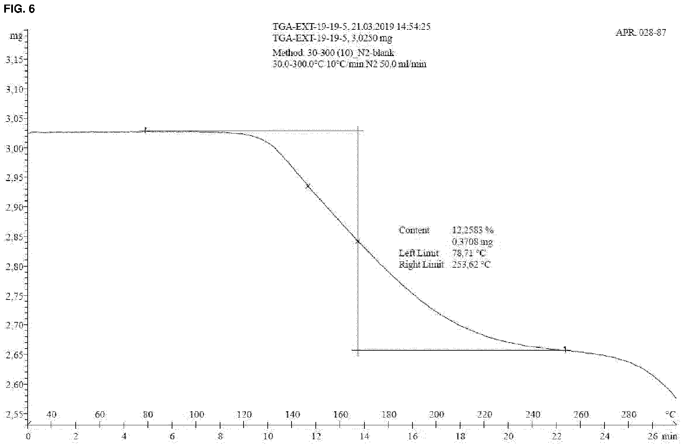

The product was characterized by TGA and PXRD as it can be seen in figures 2 and 6.

### Synthesis of a 3-methyl-2-cyclohexenone solvate of a compound of formula I (apremilast)

Apremilast (12g, 26mmol) and 3-Methyl-2-cyclohexenone (36 mL, 3.0 RV) were charged into a 50mL round-bottom flask, and the mixture was stirred at RT for a 3h. Cyclohexane (36 mL, 3.0 RV) was added to the solution, and then seeded. The mixture was stirred overnight, cooled to 0-5 ºC, and stirred for 2 additional hours. The final solid was filtered and was washed with cyclohexane. The product was dried under vacuum at 45 ºC until constant weight (12.72 g).

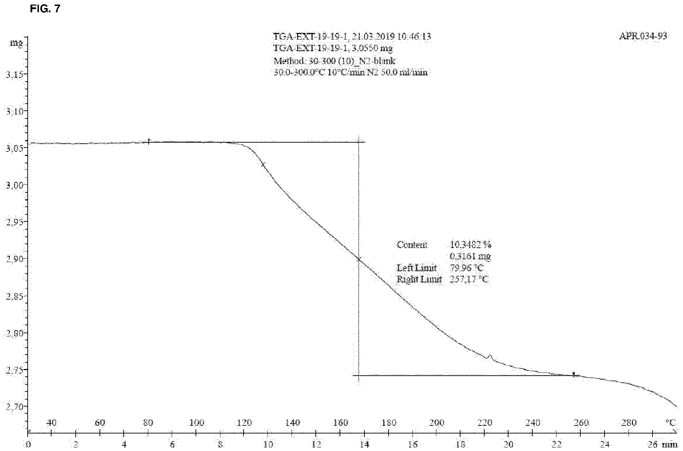

The product was characterized by TGA and PXRD as it can be seen in figures 3 and 7.

### Synthesis of a 6-methyl-5-hepten-2-one solvate of a compound of formula I (apremilast)

Apremilast (12g, 26mmol) and 6-Methyl-5-hepten-2-one (24 mL, 2.0 RV) were charged into a 50mL round-bottom flask, and the mixture was stirred at 75-80ºC overnight. Once the reaction was completed, the reaction was cooled down to RT and then to 0-5ºC and stirred for 2 h. The product was filtered and washed with MTBE (36 mL, 3.0 RV). The solid was dried under vacuum at 45 °C until constant weight, to obtain 13.11 g of the product.

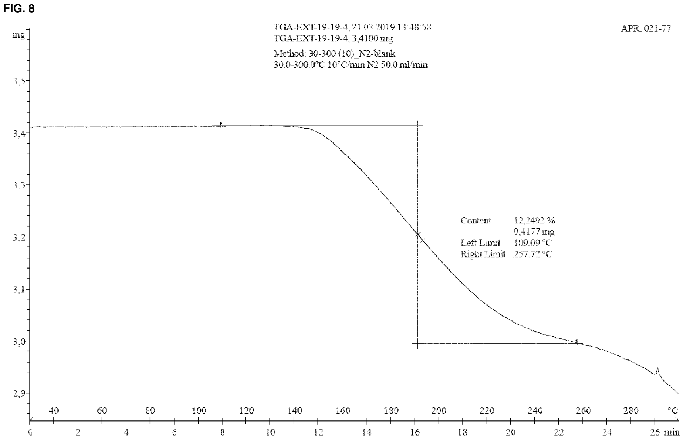

The product was characterized by TGA and PXRD as it can be seen in figures 4 and 8.

## Claims

1. A process for manufacturing the compound of formula I or a salt or solvate thereof, 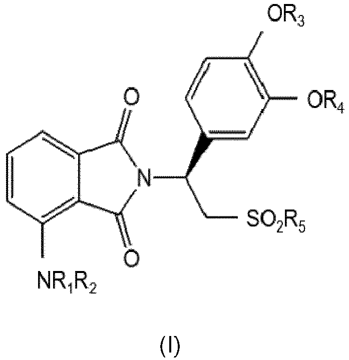 wherein
R₁ and R₂ are independently selected from H, C₁-C₈ alkyl, C₃-C₆ cycloalkyl, aryl or C=OR₆ wherein R₆ is independently selected from H, C₁-C₈ alkyl, C₃-C₆ cycloalkyl, and aryl,
R₃ and R₄ are independently selected from H, C₁-C₈ alkyl, C₃-C₆ cycloalkyl, and aryl,
R₅ is C₁-C₈ alkyl, C₃-C₆ cycloalkyl, and aryl,
the process comprising the following step:
(a) reacting the compound of formula II or a salt thereof 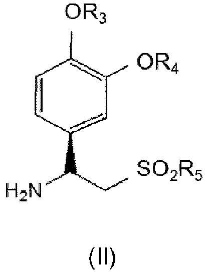 with the compound of formula III.

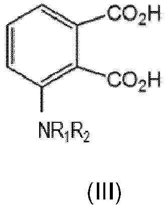

2. The process of claim 1, wherein R₁ is H, R₂ is acetyl, R₃ is methyl, R₄ is ethyl and R₅ is methyl.

3. The process of any one of claims 1 or 2, wherein compound III is activated by a coupling agent, preferably an acid activating agent, more preferably selected from carbonyldiimidazole (CDI), thionyl chloride, dicyclohexyl carbodiimide (DCC), 1-Ethyl-3-(3-dimethylaminopropyl)carbodiimide (EDC), hydroxybenzotriazole (HOBt), Hexafluorophosphate Benzotriazole Tetramethyl Uronium (HBTU), Hexafluorophosphate Azabenzotriazole Tetramethyl Uronium (HATU), 6-chloro-1-((dimethylamino)(morpholino)-methylene)-1 H-benzotriazolium hexafluorophosphate 3-oxide (HDMC), preferably is activated by CDI.

4. The process of any one of claims 1 to 3, wherein the solvent is an organic solvent, preferably selected from acetonitrile (ACN), dichloromethane (DCM), tetrahydrofuran (THF), 2-methyltetrahydrofuran, toluene and ethanol, more preferably is ACN.

5. The process of any one of claims 1 to 4, step (a) is performed under heating to reflux.

6. The process of any one of claims 1 to 5, further comprising (b) precipitating the compound of formula I.

7. The process of claim 6, wherein compound I precipitates from a mixture of the organic solvent, preferably ACN, and water, more preferably from an about 1:1 to 10:1 mix of ACN and water, even more preferably from an about 6:1 mix of ACN and water.

8. The process of any one of claims 1 to 7, wherein compound III is synthesized from compound IV.

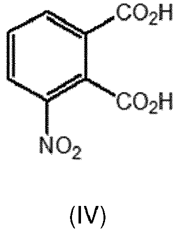

9. The process of claim 8 wherein compound IV is transformed into compound V before transforming into compound III.

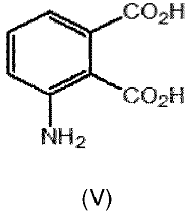

10. The process of any one of claims 8 or 9, comprising the use of a catalyst and a reducing agent, the catalyst being preferably selected from Pd, Pt, Rh, Ru, Raney nickel, Fe, Sn and Zn in HCI, and the reducing agent being preferably selected from formic acid, formates selected from ammonium, potassium and sodium formates, H₂, sodium borohydride, polimethylhidrosiloxane (PMHS) and triethylsilane (TES).

11. The process of claim 10, wherein the amount of catalyst is from about 1 to 10 % by weight, preferably from about 1.5 to 2.5 % by weight of catalyst in respect of the weight of compound IV; and wherein preferably formic acid is used as reducing agent and Pd is used as catalyst, preferably Pd/C.

12. The process of any one of claims 8 to 11, wherein compound III synthesis is carried out at a temperature below 60 ºC, preferably below 50 ºC, more preferably between 35 and 45 ºC.

13. The process of any one of claims 1 to 12, further comprising providing a solution of the compound of formula I in a solvent selected from anisaldehyde, 3-phenylpropanaldehyde, 3-methyl-2-cyclohexenone and 6-methyl-5-hepten-2-one and isolating the respective solvate of the compound of formula I.

14. A crystalline anisaldehyde solvate of apremilast, a crystalline 3-phenylpropanaldehyde solvate of apremilast, a crystalline 3-methyl-2-cyclohexenone solvate of apremilast, or a crystalline 6-methyl-5-hepten-2-one solvate of apremilast obtainable by the process according to claims 1 to 13.

15. A pharmaceutical composition comprising the compound of formula (I) or any of the solvates obtained by the process of any one of claims 1 to 13 and at least one pharmaceutically acceptable excipient.

16. A crystalline anisaldehyde solvate of apremilast, having an X-ray powder diffractogram comprising at least the peaks at diffraction 2-theta angle 7.40 ± 0.2°, 11.15 ± 0.2°, and 26.10 ± 0.2º.

17. The crystalline anisaldehyde solvate of apremilast of claim 16, having an X-ray powder diffractogram comprising at least the peaks at diffraction 2-theta angle 7.40 ± 0.2°, 11.15 ± 0.2º, 26.10 ± 0.2º, 17.59 ± 0.2º, and 16.25 ± 0.2º.

18. The crystalline anisaldehyde solvate of apremilast of any one of claims 16 or 17, having an X-ray powder diffractogram pattern as shown in figure 1.

19. A crystalline 3-phenylpropanaldehyde solvate of apremilast, having an X-ray powder diffractogram comprising at least the peaks at diffraction 2-theta angle 7.31 ± 0.2°, 11.08 ± 0.2°, 25.96 ± 0.2°, and 11.75 ± 0.2°.

20. The crystalline 3-phenylpropanaldehyde solvate of apremilast of claim 19, having an X-ray powder diffractogram comprising at least the peaks at diffraction 2-theta angle 7.31 ± 0.2º, 11.08 ± 0.2º, 25.96 ± 0.2º, 11.75 ± 0.2º, 16.14 ± 0.2º, 17.47 ± 0.2º and 25.08 ± 0.2°.

21. The crystalline 3-phenylpropanaldehyde solvate of apremilast of any one of claims 19 or 20, having an X-ray powder diffractogram pattern as shown in figure 2.

22. A crystalline 3-methyl-2-cyclohexenone solvate of apremilast, having an X-ray powder diffractogram comprising at least the peaks at diffraction 2-theta angle 7.41 ± 0.2°, 11.17 ± 0.2º, 26.16 ± 0.2º, and 17.61 ± 0.2°.

23. The crystalline 3-methyl-2-cyclohexenone solvate of apremilast of claim 22, having an X-ray powder diffractogram comprising at least the peaks at diffraction 2-theta angle 7.41 ± 0.2º, 11.17 ± 0.2º, 26.16 ± 0.2º, 17.61 ± 0.2º, 22.33 ± 0.2º, 24.60 ± 0.2º, 21.21 ± 0.2º, 16.29 ± 0.2º, 25.29 ± 0.2º, and 23.30 ± 0.2º.

24. The crystalline 3-methyl-2-cyclohexenone solvate of apremilast of any one of claims 22 or 23, having an X-ray powder diffractogram pattern as shown in figure 3.

25. A crystalline 6-methyl-5-hepten-2-one solvate of apremilast, having an X-ray powder diffractogram comprising at least the peaks at diffraction 2-theta angle 11.25 ± 0.2°, 7.40 ± 0.2º, 26.10 ± 0.2º, 17.61 ± 0.2º and 17.80 ± 0.2º.

26. The crystalline 6-methyl-5-hepten-2-one solvate of apremilast of claim 25, having an X-ray powder diffractogram comprising at least the peaks at diffraction 2-theta angle 11.25 ± 0.2º, 7.40 ± 0.2º, 26.10 ± 0.2º, 11.99 ± 0.2º, 16.27 ± 0.2º, 17.61 ± 0.2º and 17.80 ±0.2º.

27. The crystalline 6-methyl-5-hepten-2-one solvate of apremilast of any one of claims 25 or 26, having an X-ray powder diffractogram pattern as shown in figure 4.
